# EUROPEAN PATENT APPLICATION

(11) **EP 2 363 077 A1**
(43) Date of publication of application: **07.09.2011**
(21) Application number: 11250253.9
(22) Date of filing: 04.03.2011
(51) Int. Cl.: A61B 17/00, A61B 19/00

(54) **Surgical instrument with integrated wireless camera**

(30) Priority: 05.03.2010 US 310803 P; 11.02.2011 US 25186
(71) Applicant: Tyco Healthcare Group LP, New Haven, CT 06511 (US)
(72) Inventor: Ma, Youg, Cheshire, CT 06410 (US); Power, James, Madison, CT 06443 (US)
(74) Representative: Soames, Candida Jane

(57) **Abstract**

A surgical instrument (100) including an integrated wireless camera includes a handle assembly (10) housing control circuitry (50). An elongated shaft (20) extends from the handle assembly and includes an end effector disposed at a distal end thereof. An image sensor assembly (40) is disposed toward the distal end of the elongated shaft and is electrically coupled to the control circuitry. The image sensor assembly is configured to convert an optical image into an electrical signal and communicate the electrical signal to the control circuitry. The control circuitry is configured to process the signal and wirelessly transmit the processed signal to a wireless receiver positioned remote of the surgical instrument.

## Description

### CROSS REFERENCE TO RELATED APPLICATION

The present application claims the benefit of and priority to U.S. Provisional Application Serial No. 61/310,803 filed on March 5, 2010, the entire contents of which are incorporated herein by reference.

### BACKGROUND

### Technical Field

The present disclosure relates to a wireless camera, and more particularly, to a surgical instrument including an integrated wireless video camera for use in laparoscopic surgeries.

### Background of Related Art

Due to recent advancements in minimally invasive, or laparoscopic surgical technology, the number of surgeries capable of being performed laparoscopicly has greatly increased. Laparoscopic surgical procedures are minimally invasive procedures in which operations are carried out within the body by means of elongated instruments inserted through small incisions in the body. The incisions are typically created by a tissue piercing instrument such as a trocar. Laparoscopic instruments are inserted into the patient through a cannula or port which maintains the incision opening in the body during the procedure.

Laparoscopic procedures are desirable in that they allow for quicker recovery time and shorter hospital stays as compared to open surgical procedures. Laparoscopic procedures also leave minimal scarring (both internally and externally) and reduce patient discomfort during the recovery period.

However, because the interior dimensions of the cannulas and/or access ports used in laparoscopic procedures are necessarily small, only elongated, small diametered instrumentation may be used to access the internal body cavities and organs. Visibility into the surgical site is also limited, if not completely occluded.

Accordingly, it would be advantageous to provide a surgical instrument for use in laparoscopic procedures which includes an integrated wireless camera capable of providing the surgeon with a real-time video image of the surgical site. Further, a surgical instrument including an integrated wireless camera would free up and/or reduce the number of access ports required for a particular procedure since a separate port for an endoscope would no longer be required.

### SUMMARY

In accordance with the present disclosure, a surgical instrument having an integrated wireless camera is provided. The surgical instrument includes a handle assembly housing control circuitry, e.g., a processing component and a wireless transmitter. An elongated shaft extends from the handle assembly and includes an end effector disposed at a distal end thereof. An image sensor assembly, e.g., a lens and an image sensor, is disposed toward the distal end of the elongated shaft. The image sensor assembly is electrically coupled to the control circuitry disposed within the handle. In operation, the image sensor assembly converts an optical image to an electrical signal. The electrical signal is communicated to the control circuitry where it is processed, e.g., converted from analog to digital or from digital to analog. The processed signal is wirelessly transmitted to a wireless receiver positioned remote of the surgical instrument.

In one embodiment, the surgical instrument further includes an antenna mounted on the handle assembly. Alternatively, the antenna may be disposed within the handle. The antenna is configured to facilitate the transmission of the processed signal from the wireless transmitter to the wireless receiver.

In another embodiment, the wireless receiver is coupled to a video display for displaying the transmitted signal as a video image.

In yet another embodiment, the image sensor is a CCD image sensor. Alternatively, the image sensor may be a CMOS image sensor.

In still another embodiment, the surgical instrument further includes a battery disposed within the handle assembly. The battery is configured to power the control circuitry and the image sensor.

In still yet another embodiment, the electrical signal produced by the image sensor is an analog signal. Alternatively, the electrical signal may be a digital signal. Accordingly, the control circuitry may be configured to convert the electrical signal from an analog to a digital signal or, from a digital to an analog signal. More specifically, where the electrical signal is an analog signal, the control circuitry may convert the signal into a digital signal. Where the electrical signal is a digital signal, the control circuitry may convert the signal into an analog signal.

In yet another embodiment, the control circuitry is configured to broadcast the analog signal (or converted analog signal) to the wireless receiver. The analog signal may be synthesized with a carrier frequency of, for example, 2.4GHz to be broadcasted wirelessly to the wireless receiver. In such an embodiment, the wireless receiver can then decouple the signal and feed the signal to a video display for displaying the signal as a video image.

In still yet another embodiment, the control circuitry is configured to wirelessly transmit the digital signal (or digitized signal) according to a Bluetooth, Wi-Fi, Zigbee, or other standard protocol. Thus, a standard wireless receiver, e.g. a Bluetooth, Wi-Fi, or Zigbee receiver, may be used to decouple and feed the signal to a video display for displaying the signal as a video image.

### BRIEF DESCRIPTION OF THE DRAWINGS

Various embodiments of the subject instrument are described herein with reference to the drawings wherein:

Fig. 1 is a perspective view of a surgical instrument having an integrated wireless camera, and a wireless receiver coupled to a video display in accordance with the present disclosure; and

Fig. 2 is a cut-away view of a handle assembly of the surgical instrument of Fig. 1 showing the internal components of the handle assembly.

### DETAILED DESCRIPTION

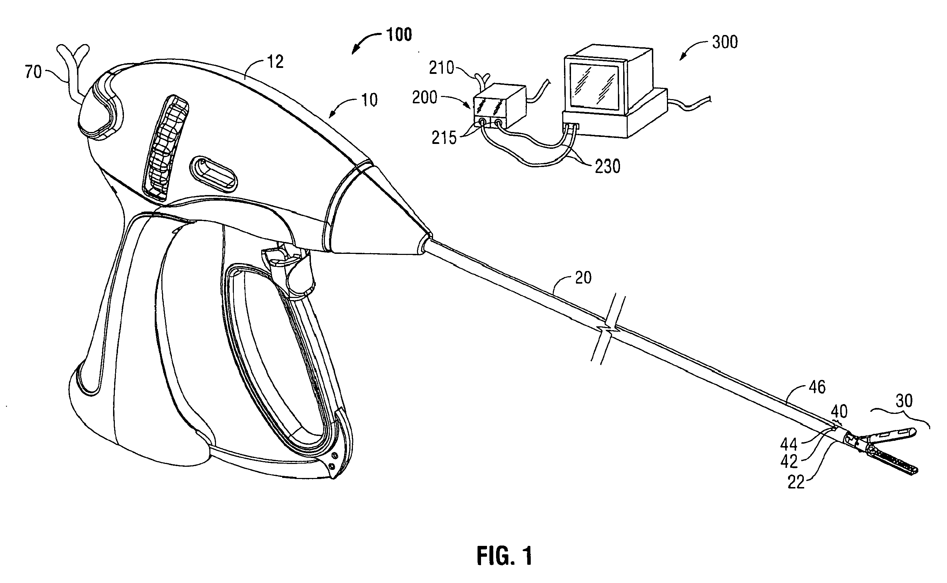

Turning now to Fig. 1, a surgical instrument including an integrated wireless camera is shown designated by reference numeral 100. Surgical instrument 100 generally includes a handle assembly 10, an elongated shaft 20 extending from the handle assembly 10 and an end effector 30 disposed at a distal end 22 of the elongated shaft 20. As will be described in greater detail hereinbelow, surgical instrument 100 includes an integrated wireless camera configured to wirelessly transmit a signal to a wireless receiver 200. Wireless receiver 200 is configured to decouple the signal and feed the signal to a video display 300 to display the signal as a video image. As can be appreciated, a surgical instrument, e.g., surgical instrument 100, including an integrated wireless camera is particularly advantageous for use in minimally invasive as well as open surgical procedures. More specifically, a surgical instrument 100 including an integrated wireless camera allows a surgeon as well as the surgical team to view a real-time image of the surgical site on a video display, without the need for additional incisions or larger incisions to allow camera to be inserted into the body. The wireless feature eliminates may of the difficulties associated with wired surgical cameras, e.g., limited maneuverability and the potential for tangling and/or disconnection. The fully integrated wireless camera is also advantageous in that it is disposed within the surgical instrument 100 without requiring the shaft 20 to be expanded to accommodate the components of the camera. As can be appreciated, this feature is particularly desirable for minimally invasive procedures, where access ports into the body are relatively small in diameter. Further, although surgical instrument 100 is shown as a surgical forceps 100, it is envisioned that the present disclosure be adaptable to numerous surgical instruments, thus providing a wireless integrated camera for use in various minimally invasive or open surgical procedures.

With continued reference to Fig. 1, and as mentioned above, surgical instrument 100 includes an elongated shaft 20 extending from handle assembly 10. During a minimally invasive, or laparoscopic procedure, for example, elongated shaft 20 may be inserted through an access port (not shown), or opening in tissue, to position the end effector 30 within an internal body cavity. As can be appreciated, visibility into the surgical site may be limited, or completely occluded due to the presence of the elongated shaft 20 disposed through the access port (not shown), or opening in tissue. Accordingly, as shown in Fig. 1, an image sensor assembly 40 is positioned toward a distal end 22 of elongated shaft 20. It is envisioned that image sensor assembly 40 may be disposed at various positions toward the distal end 22 of elongated shaft 20, or may be disposed on the end effector 30, depending on the dimensions of the surgical instrument, the particular procedure to be performed and/or the desired field of view.

The image sensor assembly 40 includes a lens 42 and an image sensor 44. The lens 42 is configured to project an optical image onto the image sensor 44. To this end, the lens 42 (or group of lenses) may be configured to focus, magnify, or otherwise modify the optical image projected onto the image sensor 44. The image sensor 44 is configured to convert the optical image into an electrical signal. The image sensor 44 may be a CCD image sensor, a CMOS image sensor, or any other suitable image sensor as is known in the art. Further, the image sensor 44 may be either a digital or an analog image sensor and, thus, may be configured to produce either a digital or an analog signal.

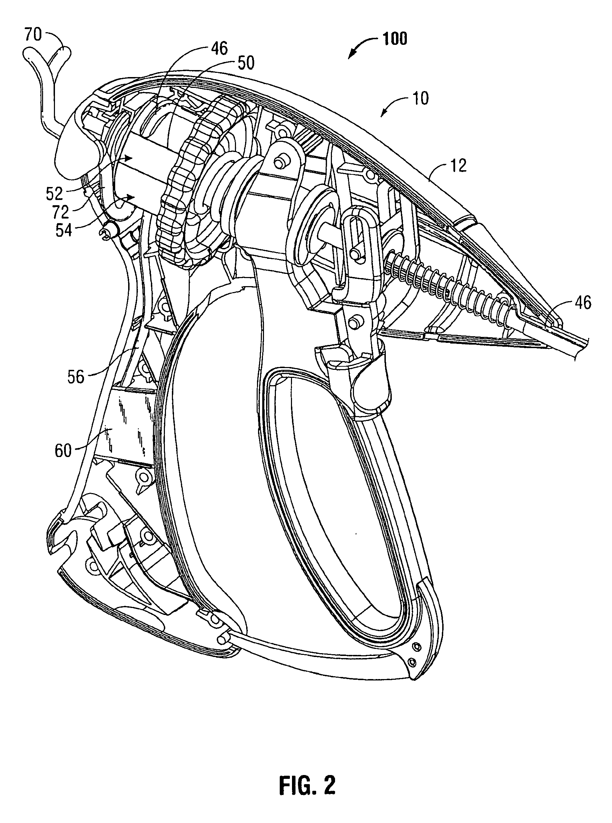

As shown in Fig. 1, the image sensor 44 is electrically coupled to an insulated wire, or bundle of wires 46 extending from the image sensor assembly 40 proximally through the shaft 20 and into the handle assembly 10 of surgical instrument 100. Bundle of wires 46 is configured to transmit the electrical signal produced by the image sensor 44 through the shaft 20 and to the control circuitry 50 (Fig. 2) disposed within the handle assembly 10, as will be described in greater detail hereinbelow. Bundle of wires 46 is also configured to transfer power to the image sensor 44 from a battery 60 (Fig. 2) disposed within the handle assembly 10.

With reference now to Fig. 2, handle assembly 10 is shown with a portion of handle housing 12 removed to show the internal components of handle assembly 10. As shown, bundle of wires 46 extends proximally through the shaft 20 into the handle assembly 10, as mentioned above, coupling image sensor 44 to the control circuitry 50. Wire 56 provides power to the control circuitry 50 and ultimately connects to wire bundle 46 to power the image sensor 44 from the battery 60.

Control circuitry 50 includes a processing component 52 and a wireless transmitter 54. More specifically, the signal produced by the image sensor 44 is communicated to the processing component 52, which processes the signal, e.g., converts the signal from analog to digital or digital to analog, or modulates the signal. In one embodiment, for example, the image sensor 44 communicates an analog signal to the processing component 52 which, in turn, synthesizes the signal with a carrier frequency, e.g., 2.4 GHz, and communicates the modulated signal to the wireless transmitter 54. Where the signal is a digital signal, the processing component 52 may be configured to first convert the signal to analog before modulating the signal and transmitting the signal to the wireless transmitter 54. In another embodiment, for example, the image sensor 44 communicates a digital signal to the processing component 52. The processing component 52 digitally modulates the signal and communicates the signal to the wireless transmitter 54. If the signal from the image sensor 44 is analog, the processing component may be configured to digitize the signal before communicating the signal to the wireless transmitter 54.

The wireless transmitter 54 is configured to wirelessly transmit, or broadcast the processed signal to the wireless receiver 200. As mentioned above, in some embodiments, the signal is analog, or converted to analog, and modulated with a carrier frequency, e.g. 2.4 GHz, by the processing component 52. Accordingly, the wireless transmitter 54 may be configured to broadcast the modulated analog signal to the wireless receiver 200. In other embodiments, where the signal is digital, or digitized, and modulated by the processing component 52, the wireless transmitter 54 may be configured according to a standard protocol, e.g., Bluetooth, Wi-Fi, or Zigbee. Thus, the wireless transmitter 54 may be a standard, off-the-shelf product. Alternatively, any other suitable wireless transmitter 54, standard or proprietary, may be used. As shown in Fig. 2, an antenna 70 coupled to the wireless transmitter 54 via cable 72 may extend from the handle assembly 10 to facilitate transmission of the signal to the wireless receiver 200. Although antenna 70 is shown extending from handle assembly 10, it is also envisioned that antenna 70 be configured as a low profile antenna protruding minimally from handle assembly 10. Further, antenna 70 may be internally disposed within handle assembly 10.

Referring now to Fig. 1 in conjunction with Fig. 2, the wireless transmitter 54 and antenna 70, as mentioned above, are configured to transmit the signal wirelessly to the wireless receiver 200. It is envisioned that the wireless receiver 200 also include an antenna 210 to facilitate reception of the signal from the wireless transmitter 54. It is further envisioned that the wireless transmitter 54 and wireless receiver 200 have a working range suitable for use in an operating room or other surgical setting. In other words, it is envisioned that the wireless transmitter 54 disposed within the surgical instrument 100 be capable of communication with the remote wireless receiver 200 throughout the entire surgical procedure, as the instrument 100 is maneuvered during the course of the procedure.

The wireless receiver 200 may be a standard wireless receiver, e.g., a Bluetooth, Wi-Fi, Zigbee, or other off-the-shelf product according to the wireless transmitter 54, or alternatively, may be specifically configured according to the specifications of the non-standard, or proprietary wireless transmitter 54 disposed within the surgical instrument 100. In either embodiment, the wireless receiver 200 is configured to decouple, or demodulate, the signal and communicate the signal to the video monitor 300. The wireless receiver 200 may include standard electrical connections 215 such that the wireless receiver 200 may be coupled, e.g., via cables 230, to any standard video monitor 300. The video monitor 300 displays the signal as a video image.

With reference once again to Fig. 2, the battery, or battery pack 60 may be any standard battery as known in the art for powering the control circuitry 50 and the image sensor 44 of the integrated wireless camera. It is also envisioned, where the surgical instrument 100 is a battery powered instrument, that both the surgical instrument 100 and the integrated wireless camera be powered from the battery pack 60 such that the surgical instrument 100 may be fully wireless, allowing for uninhibited movement of the surgical instrument 100 during the surgical procedure. Further, the battery 60 may be replaceable and/or rechargeable.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical instrument including an integrated wireless camera, the surgical instrument comprising:
   a handle assembly housing control circuitry;
   an elongated shaft extending from the handle assembly, the elongated shaft having an end effector disposed at a distal end thereof;
   an image sensor assembly disposed toward the distal end of the elongated shaft, the image sensor assembly electrically coupled to the control circuitry; and
   wherein the image sensor assembly is configured to convert an optical image into an electrical signal and communicate the electrical signal to the control circuitry, the control circuitry configured to process the signal and wirelessly transmit the processed signal to a wireless receiver positioned remote of the surgical instrument.
2. The surgical instrument according to paragraph 1, further comprising an antenna mounted on the handle assembly, the antenna configured to facilitate wireless transmission of the processed signal from the control circuitry to the wireless receiver.
3. The surgical instrument according to paragraph 1, wherein the wireless receiver is coupled to a video display for displaying the transmitted signal as a video image.
4. The surgical instrument according to paragraph 1, wherein the image sensor assembly includes one of a CCD image sensor and a CMOS image sensor.
5. The surgical instrument according to paragraph 1, further comprising a battery disposed within the handle assembly for powering the image sensor assembly and the control circuitry.
6. The surgical instrument according to paragraph 1, wherein the electrical signal produced by the image sensor assembly is one of an analog signal and a digital signal.
7. The surgical instrument according to paragraph 1, wherein the control circuitry is configured to convert the electrical signal from the image sensor assembly into one of an analog and a digital signal.
8. The surgical instrument according to paragraph 1, wherein the control circuitry is configured to broadcast an analog signal to the wireless receiver.
9. The surgical instrument according to paragraph 8, wherein the analog signal is synthesized with a carrier frequency of 2.4GHz and broadcast to the wireless receiver.
10. The surgical instrument according to paragraph 1, wherein the control circuitry is configured to transmit the signal to the wireless receiver according to one of a Bluetooth, a Wi-Fi, and a Zigbee protocol.

## Claims

1. A surgical instrument including an integrated wireless camera, the surgical instrument comprising:
a handle assembly housing control circuitry;
an elongated shaft extending from the handle assembly, the elongated shaft having an end effector disposed at a distal end thereof;
an image sensor assembly disposed toward the distal end of the elongated shaft, the image sensor assembly electrically coupled to the control circuitry; and
wherein the image sensor assembly is configured to convert an optical image into an electrical signal and communicate the electrical signal to the control circuitry, the control circuitry configured to process the signal and wirelessly transmit the processed signal to a wireless receiver positioned remote of the surgical instrument.

2. The surgical instrument according to claim 1, further comprising an antenna mounted on the handle assembly, the antenna configured to facilitate wireless transmission of the processed signal from the control circuitry to the wireless receiver.

3. The surgical instrument according to claim 1 or claim 2, wherein the wireless receiver is coupled to a video display for displaying the transmitted signal as a video image.

4. The surgical instrument according to any preceding claim, wherein the image sensor assembly includes one of a CCD image sensor and a CMOS image sensor.

5. The surgical instrument according to any preceding claim, further comprising a battery disposed within the handle assembly for powering the image sensor assembly and the control circuitry.

6. The surgical instrument according to any preceding claim, wherein the electrical signal produced by the image sensor assembly is one of an analog signal and a digital signal.

7. The surgical instrument according to any preceding claim, wherein the control circuitry is configured to convert the electrical signal from the image sensor assembly into one of an analog and a digital signal.

8. The surgical instrument according to any preceding claim, wherein the control circuitry is configured to broadcast an analog signal to the wireless receiver.

9. The surgical instrument according to claim 8, wherein the analog signal is synthesized with a carrier frequency of 2.4GHz and broadcast to the wireless receiver.

10. The surgical instrument according to any preceding claim, wherein the control circuitry is configured to transmit the signal to the wireless receiver according to one of a Bluetooth, a Wi-Fi, and a Zigbee protocol.
